(19) Europäisches Patentamt
European Patent Office
Office européen des brevets

(11) EP 0 549 742 B1

(12) **EUROPEAN PATENT SPECIFICATION**

(45) Date of publication and mention
of the grant of the patent:
**07.04.2004 Bulletin 2004/15**

(51) Int Cl.7: **A61K 38/57**, A61P 15/00,
A61P 31/18

(21) Application number: **92911432.0**

(22) Date of filing: **02.06.1992**

(86) International application number:
**PCT/EP1992/001223**

(87) International publication number:
**WO 1992/021373 (10.12.1992 Gazette 1992/31)**

(54) **USE OF CANPs-INHIBITORS IN PHARMACEUTICAL PREPARATIONS**

Verwendung von CANP-Inhibitoren in pharmazeutischen Zubereitungen

UTILISATION D'INHIBITEURS DE PROTEASES NEUTRES ACTIVEES PAR LE CALCIUM DANS DES PREPARATIONS PHARMACEUTIQUES

(84) Designated Contracting States:
**AT BE CH DE DK ES FR GB GR IT LI LU MC NL SE**

(30) Priority: **03.06.1991 GR 10100238**

(43) Date of publication of application:
**07.07.1993 Bulletin 1993/27**

(73) Proprietor: **LOGOTHETOU-RELLA, Helen
GR-153 42 Athens (GR)**

(72) Inventor: **LOGOTHETOU-RELLA, Helen
GR-153 42 Athens (GR)**

(74) Representative: **Wallace, Sheila Jane
Lloyd Wise
Commonwealth House,
1-19 New Oxford Street
London WC1A 1LW (GB)**

(56) References cited:
**WO-A-90/00401**

- **BIOLOGICAL ABSTRACTS vol. 68 , 1979,
  Philadelphia, PA, US; abstract no. 16423,
  NISHIURA, IWAO ET AL. 'THE ROLE OF
  INTRACELLULAR PROTEASES IN BRAIN
  TUMOR.'**

Remarks:
The file contains technical information submitted after the application was filed and not included in this specification

## Description

FIELD OF THE INVENTION

The present invention relates to the use of specific inhibitors of Calcium Activated Neutral Proteases (CANPs) for the manufacture of a pharmaceutical preparation for the treatment of tumours, especially cancer, viral diseases, AIDS, and as a contraceptive.

BACKGROUND OF THE INVENTION

One of the most lethal properties of malignant cells is their ability to infiltrate normal tissues and to metastasize to distant areas. The normal connective tissues consist of cells embedded in an extracellular matrix containing glycoproteins, collagen, elastin and proteo-glycans. There have been made suggestions that tumour associated histolytic enzymes may aid in the invasive process by removal of the matrix protein (Hart, I. et al., 1980, JNCI 64:891). Several studies have concentrated on this aspect of tumour cell biology, and increased protease production has been observed with many transformed cells (Jones P.A. and Declerk Y.A., 1980, Cancer res. 40:3222).

m- and μ-calcium-activated neutral proteases (CANPs), also known as calpain I and II, are typical intracellular cysteine proteinases of higher animals. They have been presumed to participate in various cellular functions mediated by $Ca^{2+}$, but their precise function are not yet clear. CANPs hydrolyse proteins of limited classes in vitro, including epidermal growth factor receptor, platelet derived growth factor receptor and protein kinase C. They appear to be involved in regulating the turnover and degradation of muscle myofibrillar proteins and neuronal cytoskeletal elements, suggesting that CANPs are involved in essential cellular functions (Murachi T., 1983, Trends. Biochem. Sci. 8, 167-169).

In studying the role of CANPs in the above processes, several exogenous inhibitors of this enzyme have been utilized, for example leupeptin, a peptide of the structure N-acetyl-L-leucyl-L-leucyl-L-arginal, and E64, an epoxy compound of the peptide structure L-trans-3-carboxy-oxiran-2-carbonyl-L-leucylagmatine, both specific inhibitors of thiol proteases.

Leupeptin and E64 have been proposed for use in the treatment of Duchenne Muscular Dystrophy (Hollenberg Sher J. et al., 1981, Proc. Natl. Acad. Sci. USA 78(12), 7742-7744, and Komatsu K. et al., 1986, Exper. Neurol. 91, 23-29). In addition, leupeptin and E64 have been used as pharmaceutical preparations in promoting synapse formation and innervation of muscle fibers (PCT-application WO-A-9000 401, 1990, University College, London).

E64 arrests human epidermoid carcinoma A431 cells at mitotic metaphase (Shoji-Kasai, Y. et al., 1988, Proc. Natl. Acad. Sci. USA 85, 146-150.)

In the field of cancer, only leupeptin has been used and found to inhibit the in vitro cell growth of rat brain cells only: (Nishiura I. et al., 1979, Neurol. Med. Chir. (Tokyo) 19(1), 1-8).

It is believed that the small molecules leupeptin and E64 may penetrate cell membranes and enter nerve terminals and cells and thus inhibit calcium activated proteinase (Nishiura I. et al., 1979, Neurol. Med.-Chir. 19(1), 1-8 and PCT-application WO-A-9 000 401, 1990, University College, London).

This suggested that leupeptin could be used for therapeutic purposes. However, such treatment has not been found especially successful. This is because CANPs are not inhibited (Mehdi S. et al., 1988, Biochem. and Biophys. Res. Comm. 157(3) 1117-1123) or only partially inhibited by leupeptin (Tsuji S. and Imahori K, 1981, J. Biochem. 1990, 233-240). In addition, the substrate in malignancies upon which the inhibitors of CANPs act were unknown until now. Moreover, as will be reported later, leupeptin and E64 used in tests according to the present invention did not inhibit malignant cell growth.

It has been published that malignant cells in culture from human invasive urothelial carcinoma form tumour nodules and glycosaminoglycan membranous sacs (GSG) with membrane extensions intracellularly as well as extracellularly (Logothetou-Rella, H. et al., 1988a, Europ. Urol. 14(i), 61-64 and 65-71). The same observations were made in human trophoblast cell cultures (Logothetou-Rella, H. et al., 1989, Histol. Histopath. 4:367-374), while they were not found in human normal urothelial cells in culture (Logothetou-Rella, H. et al., 1988, Europ. Urol. 15, 259-263). The participation of GSG has also been reported in capillary formation which is enhanced in tumours in vivo (Logothetou-Rella, H. et al., 1990, Histol. Histopath. 5:55-64).

The characteristic extracellular matrix (GSG) of malignant and embryonic cells is PAS and PAS-diastase positive, identified by Papanicolaou stain by its light green colour (EA colour) and smooth, to fibrillar translucent texture. GSG in malignant cells is distributed and accumulated in intracellular and extracellular membranous sacs. The membranous GSG sacs give rise to membrane extensions which form channels through which the green GSG is passed from the inside to the outside of the cell, enhance tumour nodule formation and invade other cells in vitro.

OBJECT OF THE INVENTION

Surprisingly a new mechanism of cell to cell invasion and substrate (GSG bound CANP) formation, common e.g. in formation of tumours, viral diseases, AIDS and fertilization were found.

Moreover it was found that administering specific inhibitors of CANPs to provide an effective concentration of said inhibitors in human or animal body would inhibit the aforementioned processes.

DETAILED DESCRIPTION OF THE INVENTION

It was found that the intracellular and extracellular matrix (sac-GSG bound CANPs) produced by the interaction of malignant with normal cells in vitro and in vivo can be used as substrate for the specific inhibitors of CANPs.

The intracellular GSG bound CANPs-sacs communicate with the extracellular environment with membrane extensions and form large extracellular channels (full of substrate) or diffused matrix allowing the large molecule (approximately MW 240,000) of the inhibitors of CANPs to also enter the cells and inactivate GSG bound CANPs. The sac-GSG bound CANPs and extracellular matrix e.g. in tumours are produced by a new mechanism of cell to cell invasion related to that of viral cell infections and fertilization. The inhibitors of CANPs selectively kill malignant cells only by inactivation of the intracellular and extracellular GSG bound CANPs, a special matrix upon which viability and propagation of only malignant cells depends on.

The viral infection of cells and oocyte penetration by spermatozoa are biological phenomena which also involve cell to cell invasion, i.e. cell invasion by virus and oocyte invasion by spermatozoa, produce the same extracellular matrix (substrate) as malignant cells and require the presence of CANPs. Based on this new mechanism of cell to cell invasion, the inhibitors of CANPs or its active subunits also exhibit antiviral and contraceptive action.

A possible mechanism of the action of an inventive inhibitor on malignant cells might be e.g. the dissociation of the inhibitor tetramer, upon contact with extracellular GSG (substrate) bound CANPs, into subunits (e.g. monomers, the MW of which vary considerably and depend on the substrate used) and formation of inactive inhibitor-proteinase complex (blue hematoxylinophic granules).

Then the following events may be taking place. The inhibitor subunit produced extracellularly may diffuse through malignant cell membrane and inactivate the endogenous CANPs or the intracellular activated CANPs diffuse extracellularly towards a lower concentration gradient (after the inactivation of the extracellular activated CANPs) and get inactivated by the extracellular inhibitor monomer. Also both events might be taking place. Empty cytoplasmic vacuoles observed in inhibitor treated malignant cells support the diffusion of activated CANPs and its inactivation extracellularly.

Moreover the extracellular GSG-CANPs channels are large enough for passage of the inhibitor all the way into the GSG-CANPs sacs.

The effect of the inhibitors of CANPs on spermatozoa suggests that spermatozoa are associated with the CANPs enzyme, upon which their motility, viability and penetration ability depends. The increased sperm motility observed with high $Ca^{2+}$ concentration (Fakih et al., 1986, Fertil. Steril. 46(s), 938-944) could be achieved via the activation of sperm endogenous CANPs. The high $Ca^{2+}$ release by the egg cortex upon polyspermy prevention (Steinhardt et al., 1977, Develop. Biol. 58, 185-196) might involve the participation of the oocyte's possible inhibitors of CANPs.

The cytologic effect of the inhibitors of CANPs on semen mucin strongly documents that the active enzyme is bound to glycosaminoglycans, an apparently common substrate for both the protease and inhibitor action. Again the mechanism of action of the inhibitors on spermatozoa might be the dissociation of a certain inhibitor upon contact with mucin (substrate) bound CANPs and formation of inactive inhibitor -proteinase complex (blue hematoxylimorphic granules). The inhibitor subunit produced extracellularly after dissociation, is probably diffused through spermatozoa membrane and inactivates the endogenous CANPs or CANPs moves extracellullarly towards a lower concentration gradient after inactivation of extracellular CANPs. The inhibitors of CANPs being non-toxic to normal cells and toxic to spermatozoa seems a promising male contraceptive agent.

In a first aspect of the present invention, there is provided the use of a specific inhibitor of calcium activated neutral proteinases (CANPs) for the manufacture of a pharmaceutical preparation, for the treatment of a tumour, especially cancer.

In a second aspect of the present invention, there is provided the use of a specific inhibitor of calcium activated neutral proteinases (CANPs) for the manufacture of a pharmaceutical preparation for the treatment of a viral disease.

In a third aspect of the present invention, there is provided the use of a specific inhibitor of calcium activated neutral proteinases (CANPs) for the manufacture of a pharmaceutical preparation for the treatment of AIDS.

In a fourth aspect of the present invention, there is provided the use of a specific inhibitor of calcium activated neutral proteinases (CANPs) for the manufacture of a contraceptive.

The specific inhibitor of CANPs used in the invention are preferably endogenous native inhibitors isolated from a biological source, like erythrocytes, brain, cardia muscle, lung, spleen, liver, skeletal muscle, kidney, testis or the like, and optionally purified, but especially from rabbit skeletal or liver, comprising a tetrameric protein of MW of approximately 240,000, based on its elution from Sephadex G-200, heatstable at neutral pH, destroyed on digestion with trypsin, and dissociated into its subunits of a MW of approximately 60,000 by 0.1-1 mM CA$^{2+}$ based on SDS-polyacrylamid gel electrophoresis (as described by Melloni *et al* in Arch. of Biochem. and Biophys. Vol. 232, No. 2, 513-19, 1984). Also disclosed herein are all pharmaceutical acceptable salts thereof.

It is believed that the MWs of the active subunits of the specific inhibitor depend on the substrates (e.g. Casein, denatured globin etc.) used. Therefore, the MW of the active part of the inhibitor may be higher, e.g. approximately 150,000, or lower, e.g. approximately 15,000, as indicated above.

A preferred inhibitor isolated from rabbit skeletal is manufactured and sold by Sigma Chemical Company, St. Louis, USA, under the product number P-0787.

The inhibitors may also be produced synthetically, especially by bio- or gentechnological methods, e.g. by expression in Escherichia coli.

In the present invention the pharmaceutical preparation may be in the form of a solution, powder, injection, tablet, capsule, pellets, in a fast or sustained release form, each containing a suitable amount of a specific native or synthetic and eventually purified inhibitor or its pharmaceutically acceptable addition salts, together with well-known suitable excipients.

The inhibitors may preferably be administered to humans and warmblooded animals intramuscularly, subcutaneously, intraperitoneally or intravenously in an amount which depends on the kind and severity of the disease, the inhbitory effect of the inhibitor, the route of administration, the species to be treated, the weight and the general condition of the patient, and has in most cases finally to be decided by the responsible physician. In general the dose is between about 1 mg/kg per day and 25 mg/kg per day. However, if need be also higher doses, e.g. up to 100 mg/kg per day, may be administered.

The surprising effect of the inhibitors of CANPs have been confirmed and verified by the following tests, whereby all tests have been fulfilled also with Aprotinin (Sigma A-4 529), Trypsin-Chymotrypsin inhibitor (Sigma T-9777), Leupeptin (Sigma L-2884) and E64 (Sigma E-3132) dissolved in RPMI-1 640 with 25 mM hepes as control protease inhibitors. All inhibitor solutions were filtered through 0.22 μ Sartorius filters, dispensed in aliquots and frozen at -20°C. Fresh or thawed inhibitor solutions were used.

However, the surprising effect was only performed by the inhibitors of CANPs according to the invention. In the following tests the brownish tan powder of the inhibitor of CANPs (Sigma Chemical Company, St. Louis, USA, product number P-0787), 50 U/645 mg solid from rabbit skeletal muscle, was dissolved in 5 ml plain RPMI-1640 with 25 mM hepes (Seromed), resulting to a clear tan solution (10 U/ ml), whereby one unit (U) is that quantity of inhibitor which will reduce the activity of 1 unit of CANPs (Sigma Chemical Company, product number P 4533) by 50 % at pH 7.5 at 30°C (reaction volume = 1.8 ml, 1 cm light path). Of course, the scope of the invention shall not be reduced to the use of the concrete inhibitor used in the following examples.

EXAMPLE 1

The use of the native Inhibitor of CANP for inhibiting growth and viability of malignant cells in Vitro

Cell culture establishment

Stationary cell cultures were established from human solid tumour tissue specimens by enzymatic digestion. Malignant lung cell lines from metastatic lung carcinoma, M-cells, P-cells and B-cells have recently been characterized by the applicant. Malignant urothelial cell cultures were established from tissue specimen from patients with invasive transitional cell carcinoma. The five established urothelial malignant cell lines were designated as Pa-cells, R-cells, S-cells, Br-cells and IG-cells. Only the patient where Pa-cells derived, had received bladder intravesical infusions of anticancer drugs. Melanoma cell culture (Ha-cells) was originated from a male patient who suffered from primary rectal melanoma, metastasized at the lymph nodes of the right arm where tissue specimen was obtained.

Malignant bone marrow cells were originated from bone marrow aspirates from five (5) patients with chronic myeloid leukemia. Walker tumour rat cells were isolated from transplanted tumour tissue into Wistar rats. Normal human liver cells (L-cells) were isolated from liver tissue specimen from a male patient who underwent surgery for the removal of his gall bladder.

Normal fallopian tube cells (F-cells) were isolated from tissue specimen from a female patient who underwent total hysterectomy. Normal bladder cells (N-cells) have been characterized previously (Logothetou-Rella, H. et al., 1988, Europ. Urol. 15, 259-263). White blood cells from five healthy persons were also used as control cells.

Mice embryos harvested at the 2-cell stage were cultivated in complete Earle's balanced salt solution (EBSS supplemented with 10% foetal bovine serum and antibiotics) to the stage of hatched blastocysts. When the embryonic cells were all out, the culture was used for cytology. Amniotic ebryonic cells from five (5) pregnant women cultivated for prenatal diagnosis were also used in this study. All cell cultures were grown in complete medium, RPMI-1640 (Seromed) supplemented with 10% foetal bovine serum (Seromed), glutamine and antibiotics (Seromed), incubated at 37°C in a $CO_2$-humidified incubator. Stock cells are stored frozen in liquid nitrogen.

Cytogenetic analysis

Chromosomal analysis of M-cells, P-cells and B-cells have recently been reported (Logothetou-Rella, H. et al., 1991, J.Exper. Clin. Cancer Res., submitted for publication). Urothelial malignant, Pa-cells consisted of malignant cell clones only, with polyploidies up to 147 chromosomes and complex structural abnormalities. S-cells consisted of malignant cell clone with regular tetraploidies, up to 20% of the cell population and 80% normal cell clone. Br-cells consisted of normal and malignant cell clones but detailed chromosomal analysis was unsuccessful. Melanoma Ha-cells revealed only double minutes. Liver L-cells, fallopian tube F-cells, and amniotic embryonic cells were cytogenetically normal.

Two techniques were used to determine the inhibitor's cytotoxicity on tumor and normal cells.

a) Cytologic changes of cell cultures in continuous presence of the inhibitors

Seven kinds of complete medium RPMI-1640 were prepared. One was supplemented with 1 U/ml of the inhibitor of CANPs; the second with 2 mg/ml trypsin-chymotrypsin inhibitor; the third with 1 mg/ml aprotinin; the fourth with 1mg/ml leupeptin; the fifth with 1mg/ml E64; the sixth with all five inhibitors at the same concentration and the seventh complete RPMI-1640 as a control medium.

Ten glass petri-dishes (5 cm diameter) were seeded each with $1 \times 10^6$ M-cells and another ten dishes, each with $1 \times 10^6$ P-cells. Duplicate cell cultures received each kind of complete medium containing the inhibitors and control cultures containing only complete medium. The cell cultures were incubated at 37°C in a humidified $CO_2$-inhibitor for 120 hours. The culture medium was changed with a fresh one of the same kind in each case, 24 and 72 hours after culture initiation. Half of the cell cultures were fixed in 50% ethanol 72 hours and the other half 120 hours after culture initiation. All cell cultures were stained with the Papanicolaou method.

Post confluent stationary cell cultures of malignant M-cells, P-cells and normal L-cells (20 days of continuous cultivation) that had produced abundant extracellular matrix, received fresh complete medium RPMI-1640 supplemented with 1 U/ml of the inhibitor of CANPs and incubated at 37°C for 3 days, then fixed in 50% ethanol and stained with the Papanicolaou method.

Trypsin-chymotrypsin inhibitor, aprotinin, leupeptin and E64, did not affect the growth and cytology of M- and P-cells as compared to control cell cultures.

The inhibitor of CANPs caused great exfoliation of cells and extracellular matrix (ECM) in the culture medium, after 72 hours of continuous presence in cultures. All exfoliated cells were dead (according to trypan blue stain) consisting of hyperchromatic, pyknotic nuclei, little cytoplasm and nuclei with tails. On the culture dish surface, a few, countable per field, attached fibroblast-like cells remained alive, cytologically normal. All other cell culture dishes (except cases No. 1 an No. 6 which contained the inhibitor) and the control ones were full of cells and nuclear vlimma (="NV", "vlimma" = bullet; state of a parasitic cell after numerous dividing operations using a host cell, where it reaches the size of a nucleolus with a nuclear head and an attached tail resembling a small immotile spermatozoo. The production of nuclear heads, the shooting and implantation in other cells occurs mainly in culture areas, where extracellular glycosaminoglycans bound CANP and cell membranes are located. In normal cell cultures NV are not found, whereas it was observed e.g. in human solid and hematologic tumours free or during production still attached to the mother cell. NVs are end cell products of incomplete, unequal, asymmetrical division of malignant cells. Upon their production they eventually detach from their mother cell and seek a host cell at random. When NVs are implanted and incorporated in the nucleus of a normal host cell, it can be considered as a process similar to fertilization or viral infection. As a result, the host cell's genotype and phenotype is altered and behaves like a transformed cell. After many divisions, the host cell looses its cytoplasm and cannot divide itself anymore; it needs support by another host cell or extracellular matrix, thus forced to become a parasite and produce NVs.) uncountable per field, without cell exfoliation, with macroscopically apparent green, fibrillar, translucent ECM and GSG sacs. The observations were persistent after 120 hours of continuous presence of the inhibitor of CANPs in cell cultures, except that the survived fibroblast-like cells had grown up in the presence of the inhibitor of CANPs.

Post-confluent M- and P-cell cultures exhibited cells with vacuolated cytoplasm as a strain, and degenerated nuclei of different sizes with and without tails. The rounded up, detached, dead cells were holding to each other on the culture dish surface by a network of hematoxylinophilic (blue) membranes visible microscopically. The ECM and GSG sacs had disappeared. Instead large masses of hematoxylinophilic granules were present visible microscopically.

b) Liquid medium short-term culture method (Chang S.Y. et al, 1989, Eur. Urol. 16, 51-56).

The cells were detached with trypsin-EDTA (Seromed) resuspended in complete RPMI-1640 and cell counts were made using a hemocytometer. Viable counts were assessed using the 0.4% trypan blue exclusion method. The cells were then washed once with complete RPMI-1640, centrifuged at 200 g for 8 min, resuspended in complete RPMI-1640 at 30,000 - 200,000 cells per 0.5 ml medium and inoculated in polypropylene tubes as shown as follows:

| Test tube case No. | nCANP inhibitor (U/ml) | Volume (ml) | | | |
|---|---|---|---|---|---|
| | | nCANP inhibitor | Cell suspension | F.B.S.* | Complete RPMI-1640 |
| 1 | 1 | 0.1 | 0.5 | - | 0.4 |
| 2 | 2 | 0.2 | 0.5 | - | 0.3 |
| 3 | 3 | 0.3 | 0.5 | - | 0.2 |
| 4 | 4 | 0.4 | 0.5 | 0.05 | 0.05 |
| 5 | 5 | 0.5 | 0.45 | 0.05 | - |
| 6 | 6 | 0.6 | 0.35 | 0.05 | - |
| 7 | 0 | 0.0 | 0.5 | - | 0.5 |

*F.B.S. = Foetal bovine serum

Duplicate samples of cells were tested for each concentration of the inhibitor. All samples were incubated and shaken in a water bath at 37°C for one hour. Then the cells were washed twice with complete RPMI-1640 by centrifugation at 200 g for 8 min. Each rinsed cell pellet was resuspended in 1 ml complete RPMI-1640, the cells were then rendered single by gentle pipetting and were then seeded in 24-well microplates (Costar Cambridge Mass.) for a 4-day period of short term culture at 37°C under a humidified atmosphere of 5% $CO_2$. The cytotoxicity assessment was done using the dye exclusion method of 0.4% trypan blue. The degree of cytotoxicity was measured according to the following formula:

$$\text{Cytotoxicity (\%)} = 1 - \frac{\text{Number of viable cells in the experimental group}}{\text{Number of viable cells in the control group}} \times 100$$

The inhibitor of CANPs selectively killed all kinds of malignant cells tested (Table 1) while allowing normal cells within the same or separate culture to grow and propagate (Table 2). The optimum concentration of 4-5 U/ml inhibitor killed all malignant clones, while lower concentration killed lower percentage of malignant cells. Higher concentration did not alter the results. The inhibitor was not cytotoxic to normal cells including liver cells, fallopian cells and WBCs. Cytogenetic analysis of the survived cells (in mixed cell lines) after the inhibitor CANPs treatment showed normal karyotype. The inhibitor of CANPs was also cytotoxic to embryonic cells.

Table 1.

| Categories of cases with various cells tested. | | | | |
|---|---|---|---|---|
| Tissue origin | Designation | Malignant and normal clone | Malignant clone | Normal clone |
| Bladder transitional cell carcinoma | Pa-cells | | + | |
| | Br-cells | + | | |
| | S-cells | + | | |
| | IG-cells | | + | |
| | R-cells | + | | |
| Lung carcinoma | M-cells | + | | |
| | P-cells | + | | |
| | B-cells | + | | |
| Melanoma | Ha-cells | | + | |
| Chronic myeloid leukemia | BM-cells | | + | |

Table 1.   (continued)

| Categories of cases with various cells tested. | | | | |
|---|---|---|---|---|
| Tissue origin | Designation | Malignant and normal clone | Malignant clone | Normal clone |
| Walker rat tumor | W-cells | | + | |
| Normal liver | L-cells | | | + |
| Normal urothelium | N-cells | | | + |
| White blood cells (5 specimens) | WBC | | | + |
| Human amniotic embryonic cells (5 specimens) | | | | + |
| Human fallopian cells (F-cells) | | | | + |
| +: Indicates the cytogenetic state of each cell type. | | | | |

Table 2.

| Sensitivity of cells to different concentrations of the inhibitor of CANPs. | | | |
|---|---|---|---|
| Inhibitor cytotoxicity (%) | | | |
| Tested cells | Inhibitor-nCANP (U/ml) | | |
| | 1 | 4 | 5 |
| M-cells | 24 | 65 | 65 |
| P-cells | 45 | 82 | |
| B-cells | 86 | 87 | |
| Pa-cells | 24 | 99 | 100 |
| S-cells | | 34 | |
| Br-cells | | 55 | |
| R-cells | | 83 | |
| IG-cells | | 100 | |
| Ha-cells | 21 | | 100 |
| Walker tumor cells | | 100 | |
| Malignant bone marrow cells | | 88-100 | |
| N-cells | 0 | 0 | 0 |
| L-cells | 0 | 0 | 0 |
| WBCs | 0 | 0 | 0 |
| Embryonic cells | 45 | 95 | 100 |
| F-cells | 0 | 0 | 0 |

The cytotoxicity of the inhibitor in each specimen was obtained from mean of duplicate samples.

EXAMPLE 2

Use of the inhibitor of CANPs on the viability of normal and malignant urothelial tissues

Tumour (from 5 patients) and normal (from 5 persons) tissue pieces of human urothelium of 2 mm x 2 mm x 2mm size were rinsed in complete RPMI-1640, handled gently with fine forceps, immersed one piece (of each type of tissue) in complete RPMI-1640 (control) and one piece in the inhibitor solution (10 U/ml) in polypropylene tubes and incubated at 37°C for one hour in the humidified, 5% $CO_2$ incubator. All tissue pieces were then rinsed carefully in complete medium and were immersed in polypropylene tubes (1 piece/tube) containing 2 ml complete RPMI-1640, and then incubated for 4 days at 37°C. The tissue pieces were fixed in formaldehyde, embedded in paraffin and tissue sections were stained with eosin-hematoxylin. The exfoliated cells in the tubes with the malignant tissue pieces were allowed to settle in a conical polypropylene tube for 10 min, then smeared on glass slides fixed with cytospray and stained with Papanicolaou. The inhibitor of CANPs caused massive cell exfoliation of the malignant tissues. Histologic examination of the inhibitor treated malignant tissues exhibited bionecrotic to necrotic areas and large tissue areas consisting of eosinophilic extracellular matrix denuded of cells. The exfoliated cells were dead, with degenerated nuclei, and spermatozoa-like morphology, separated from each other and lacking the green ECM. The very few malignant tissue exfoliated cells, in the absence of the inhibitor of CANPs, showed compact cell masses in green ECM with indiscrete cell boundaries.

Normal urothelial tissues were kept intact after treatment with the inhibitor.

EXAMPLE 3

The use of the inhibitor of CANPs against human tumour nodule in vivo

A female patient with breast metastatic carcinoma, with extensive hepatic bone and subcutaneous metastasis, after repeated chemotherapy and radiation treatment without success, and also in a general bad health condition, approved the trial of the drug in her subcutaneous nodules. Nodules were located all over the chest and some in the abdomen. One hard nodule the size of a pea was injected in its center with 0.1 ml (1 U/0.1 ml) inhibitor of CANPs dissolved in RPMI-1640 with 25 mM hepes. Twenty four hours later the treated nodule and a nearby untreated control nodule were removed, fixed in formalin and embedded in paraffin for microscopical examination.

The patient did not show any allergic reaction 1, 4 and 24 hours after the injection. The nodule feeling was softer and slightly smaller 4 hours after the injection. Twenty four hours later the nodule was soft and reduced to half its originial size. The treated neoplasm was histologically characterized by degenerated small cellular aggregation and many degenerated single cells. Most of the cells had irregular pyknotic, karyolytic or degenerated vacuolated nuclei. Some cells showed vacuolated cytoplasm. In the outer peripheral area of the tumour there remained a few neoplastic cells with regular nuclei, fine chromatin and slender single nucleoli. The main area of tumour cell degeneration, caused by the inhibitor of CANPs, was measured approximately to a total of 3.4 mm x 2.5 mm out of the 5.2 mm x 2.5 mm main tumour section area. Neighboring sweat glands and the overlying epidermis were kept intact. There was no inflammatory reaction not even around the fissural hemorrhagic area caused by the injection. Histologic examination of the tumour nodule without treatment showed that the neoplasm was characterized by viable large columns or single strands of neoplastic cells with relatively uniform ovoid or roundish nuclei with finely stippled chromatin and slender nucleoli. Histologic picture was compatible with metastatic breast carcinoma.

EXAMPLE 4

The use of the inhibitor of CANPs against rat tumours in vivo

Two Walker tumours were excised 2 weeks following the subcutaneous implantation of tumour tissue in Wistar male rats. Tumour cell suspension for injection was prepared as described previously (Fisher E.R. and Fisher B., 1959, 12, 926-928). A group of Wistar male rats, weighing 100 g each, were injected with $10x10^6$ Walker tumour cells subcutaneously in the left foot pad. The rats were then divided into four groups, two control and two treated. Treatment was initiated when tumours had reached a measurable size of 50-100 cu.mm. The first group of rats was injected intraperitoneally, each rat with 50 U/2.5 ml (645 mg/2.5 ml) inhibitor of CANPs, once a day, for a period of 2 days (0.5 U/kg or 6.45 mg/kg rat body weight).

The second group of rats was treated intraperitoneally, twice daily for 5 days with the dose of 0.25 U/kg (3.23 mg/kg) rat body weight. Control rats were injected each with 2.5 ml Medium RPMI-1640 with 25 mM hepes. All rats were sacrificed 4 days after the last treatment for the injected legs, of the control groups, were all covered with tumours including up to the shoulder blade and accurate control tumour measurements were impossible. The tumour-legs, lymph nodes and liver from all rats were excised, fixed in formalin and embedded in paraffin for histologic studies. Tumour volumes were measured every day after the first dose, with calipers. The inhibitor of CANPs caused 50% tumour regression in the first group of treated rats and 90% in the second group. All groups (treated and control) started at time 0 without any significant difference in tumour volume.

The rats under treatment were healthy and did not show any allergic reaction or side effects to the high dose of the inhibitor originating from rabbit skeletal muscle. Histologic examination of livers of the treated rats did not show any cytotoxic effects caused by the inhibitor, as central venules were observed without necrosis or cellular damage.

Among the first treated group one rat developed metastatic abdominal focus and another one metastatic hepatic focus. The feeling of the abdominal focus disappeared 24 hours after the first dose. Histological examination showed necrosis of large carcinomatous nodule with formation of abscess, necrosis of the overlying epidermis and ulceration. The liver metastatic focus was necrotized, exhibiting necrotic material with nuclear debris in the center and remnants of carcinomatous tissue with mitoses in its periphery. The foodpad tumours of treated rats showed necrotic areas of variable size with formation of micro-abscesses. These results become more important if taken into account the aggressiveness of Walker tumor cells, (rats usually die 20 days after tranplantation).

CONCLUSION DEDUCED FROM EXAMPLE 1-4

The pharmaceutical composition, of the present invention killed all malignant cells of different chromosomal abnormalities, tissue and species origin without affecting normal cells' genotype.

The best in vitro dosage being the one containing 4-5 units of the inhibitor of CANPs per milliliter of solution.

This pharmaceutical composition was not cytotoxic to normal cells including liver and WBCs. It exhibited a broad spectrum of action on different types of human tumours. It was cytotoxic to human solid and hematologic tumour cells and even more to chemoresistant tumour cells (lung P-cells and bladder Pa-cells) of different tumour origin (embryonic cells were very sensitive to the inhibitor because of their resemblance to malignant cells).

The pharmaceutical composition of the present invention tested on rat tumours caused 50-90% regression of the main tumours, inhibited metastasis and caused necrosis of metastatic foci. It was non-immunogenic, non-toxic suitably used in a daily dose of 0.5 U/Kg, (6.5 mg/Kg) of body weight in single or divided doses. The administration is suitably continued until complete tumour regression.

EXAMPLE 5

Contraceptive action of the inhibitor of CANPs

Fertile motile sperm (after swim up test) from 10 donors, was dispensed in 4 plastic (5 ml, Falcon) test tubes each. Two test tubes received 0.4 ml sperm suspension (600,000 spermatozoa) and 0.6 ml complete EBSS. The other two test tubes each received 0.4 ml sperm suspension, 0.4 ml (10 U/ml) inhibitor of CANPs and 0.1 ml foetal bovine serum. All tubes were incubated at 37 °C for 1 hour. The spermatozoa were then washed twice with complete EBSS and centrifuged. The sperm pellets were resuspended each in 2 ml complete EBSS and incubated at 37°C for 18 hours, at which time spermatozoa were counted by the Eosin Y viability exclusion stain, smeared on glass slides, fixed in 50% ethanol and stained with Papanicolaou.

The degree of cytotoxicity caused by the inhibitor of CANPs was measured according to the following formula:

$$\text{Cytotoxicity (\%)} = 1 - \frac{\text{Number of viable inhibitor treated spermatozoa}}{\text{Number of viable spermatozoa of control samples}} \times 100$$

Inhibitor treated and non treated sperm was inoculated in postconfluent granulosa cell cultures, incubated at 37° C for 18 hours, fixed in 50% ethanol and stained with Papanicolaou. Motility counts of the inhibitor treated fertile sperm showed only 3% motile sperm, 2 hours post treatment and no motile sperm 18 hours post treatment. Eighty percent of the immotile sperm were dead (stained with eosin Y). Inhibitor treated sperm failed to penetrate granulosa cells during co-cultivation.

Cytologic examination revealed that 80% of the inhibitor treated spermatozoa, 18 hours post treatment, had coiled tail ends and clear acrosome caps. Moreover, the green fibrillar mucin present in the control samples was changed to dispersed loose masses of large blue granules in the inhibitor treated spermatozoa.

EXAMPLE 6

Antiviral action of the inhibitor of CANPs in vitro

1) On Epstein-Barr Virus (EBV)

Viral infection of cells which involves host-parasite interaction. Viruses are also vehicles of biological active DNA or RNA into host cells in order to survive and propagate, causing viral diseases to animals and humans.

In the following experiments the CANPs inhibitor was tested for its antiviral action in two cell lines. One cell line was cultured Burkitt tumour lymphoblasts (strain Raji) infected with Epstein-Barr virus (EBV) in vitro (Kottaridis et al, J. Natl. Cancer Inst. 1977, 59(1), 89-91) and the other P3HR-1 Burkitt lymphoma, EBV producing cells (Hinuma Y. et al, 1967, J. Virol. 1, 1045-1051). IgG antibodies were demonstrated by immunofluorescence (Gull labs) on EBV-infected Raji cell smears with 10% and on P3HR-1 with 20-25% of the cells showing fluorescence. For negative controls IgG-EBV negative antibodies were used.

For chemosensity testing 200,000 cells/tube were used. The results for EBV-infected Raji cells showed 22% cytotoxicity at 2 U/ml CANPs inhibitor, 97% at 4 U/ml and 100% at 5 U/ml. In the case of P3HR-1, EBV producing cells, there was 95% cytotoxicity at 2 U/ml CANPs inhibitor, 100% at 4 U/ml and 5 U/ml. Both cell lines were found free of detectable immunofluorescent IgG after treatment of cells with higher than 4 U/ml CANPs inhibitor.

Although both cell lines are sensitive to the CANPs inhibitor, because they are malignant, the disappearance of immunofluorescent IgG after treatment documents the antiviral action of the CANPs inhibitor.

2) On Human Immunodeficiency Virus Type 1 (HIV-1, AIDS Virus)

The cell line MOLT-4 (ATCC CRL 1582, from acute lymphoblastic leukemia) infected with HIV-1 (Koyanagi Y. S. et al., 1987, Science 236, 819-822 and Cann A.J. et al., 1990, J. Virol. 64 (10) 4735-4 742) was used. For chemosensitivity testing 200,000 cells/tube were treated with the inhibitor of CANPs at 4 U/ml and 10 U/ml. The results showed 97% cytotoxicity at 4 U/ml and 100% at 10 U/ml inhibitor of CANPs.

Cell smears, fixed in cold acetone, were used for immunofluorescent tests, using positive human serum containing antibodiesto HIV-1 (1:100) and antihuman IgG fluorescent conjugate (1:200 Dako corp.). For negative controls human serum negative to HIV-1 antibodies was used. Immunofluorescent HIV-1-antigen was detected in 60% of untreated MOLT-4 HIV-1 infected cell cultures. The treated MOLT-4 HIV-1 infected cells were free of detectable immunofluorescent HIV-1-antigen at concentrations of 4 U and 10 U/ml inhibitor of CANPs.

It is concluded that HIV-1 infected MOLT-4 cells are highly sensitive to the inhibitor of CANPs for they are malignant cells but the disappearance of immunofluorescent HIV-1 -antigen documents the anti-AIDS action of the inhibitor of CANPs.

## Claims

1. The use of a specific inhibitor of a calcium activated neutral proteinase (CANP) for the manufacture of a pharmaceutical preparation for the treatment of a tumour, especially cancer.

2. The use of a specific inhibitor of a calcium activated neutral proteinase (CANP) for the manufacture of a pharmaceutical preparation for the treatment of a viral disease.

3. The use of a specific inhibitor of a calcium activated neutral proteinase (CANP) for the manufacture of a pharmaceutical preparation for the treatment of AIDS.

4. The use of a specific inhibitor of a calcium activated neutral proteinase (CANP) for the manufacture of a contraceptive.

5. The use claimed in any of claims 1-4, wherein the inhibitor is an endogenous native inhibitor from a biological source, selected from erythrocytes, brain, cardia muscle, lung, spleen, liver, skeletal muscle, kidney or testis is purified, and comprises tetrameric proteins having MW of approximately 240,000, based on their elution from Sephadex G-200, which are heatstable at neutral pH; destroyed on digestion with trypsin, and dissociated into their subunits having a MW of approximately 60,000 or lower by 0.1-1 mM $Ca^{2+}$, based on SDS-polyacrylamide gel electrophoresis, or pharmaceutically acceptable addition salts thereof.

6. The use as claimed in claim 5 wherein the biological source of the inhibitor is selected from rabbit skeletal muscle or liver.

7. The use as claimed in claim 6 wherein the inhibitor is from rabbit skeletal muscle.

8. The use as claimed in claims 1-4, wherein the inhibitors of claims 5-7, or their pharmaceutically acceptable addition salts are produced synthetically.

9. The use of a specific inhibitor according to any of claims 1-8 in the preparation of a pharmaceutical preparation that is in the form of a solution, powder, injection, tablet, capsule, pellet, or in fast or sustained release form.

## Patentansprüche

1. Verwendung eines spezifischen Inhibitors einer calciumaktivierten neutralen Proteinase (CANP) für die Herstellung eines pharmazeutischen Präparats zur Behandlung eines Tumors, besonders Krebs.

2. Verwendung eines spezifischen Inhibitors einer calciumaktivierten neutralen Proteinase (CANP) für die Herstellung eines pharmazeutischen Präparats zur Behandlung einer Viruserkrankung.

3. Verwendung eines spezifischen Inhibitors einer calciumaktivierten neutralen Proteinase (CANP) für die Herstellung eines pharmazeutischen Präparats zur Behandlung von AIDS.

4. Verwendung eines spezifischen Inhibitors einer calciumaktivierten neutralen Proteinase (CANP) für die Herstellung eines Verhütungsmittels.

5. Verwendung nach einem der Ansprüche 1 bis 4, wobei der Inhibitor ein endogener nativer Inhibitor von einer biologischen Quelle ist, der ausgewählt ist aus Erythrozyten, Hirn, Herzmuskel, Lunge, Milz, Leber, Skelettmuskel, Niere oder Hoden, gereinigt ist und tetramere Proteine mit einer relativen Molekülmasse von etwa 240.000 auf der Basis ihrer Elution von Sephadex G-200 hat, die bei einem neutralen pH-Wert temperaturstabil sind; zerstört bei Verdauung mit Trypsin und zerlegt in ihre Untereinheiten mit einer relativen Molekülmasse von etwa 60.000 oder weniger durch 0,1-1 mM $Ca^{2+}$ auf der Basis von SDS-Polyacrylamidgelelektrophorese, oder pharmazeutisch akzeptable Additionssalze davon.

6. Verwendung nach Anspruch 5, wobei die biologische Quelle des Inhibitors aus Skelettmuskel oder Leber von Kaninchen ausgewählt ist.

7. Verwendung nach Anspruch 6, wobei der Inhibitor von Kaninchenskelettmuskel ist.

8. Verwendung nach den Ansprüchen 1 bis 4, wobei die Inhibitoren der Ansprüche 5-7 oder deren pharmazeutisch akzeptable Additionssalze synthetisch hergestellt werden.

9. Verwendung eines spezifischen Inhibitors nach einem der Ansprüche 1 bis 8 für die Herstellung eines pharmazeutischen Präparats, das in Lösungs-, Pulver-, Injektions-, Tabletten-, Kapsel-, Pelletform oder in einer Form für eine schnelle oder verzögerte Freigabe vorliegt.

## Revendications

1. Utilisation d'un inhibiteur spécifique d'une protéinase neutre activée par le calcium (CANP) pour la fabrication d'une préparation pharmaceutique pour le traitement d'une tumeur, en particulier le cancer.

2. Utilisation d'un inhibiteur spécifique d'une protéinase neutre activée par le calcium (CANP) pour la fabrication d'une préparation pharmaceutique pour le traitement d'une maladie virale.

3. Utilisation d'un inhibiteur spécifique d'une protéinase neutre activée par le calcium (CANP) pour la fabrication d'une préparation pharmaceutique pour le traitement du Sida.

4. Utilisation d'un inhibiteur spécifique d'une protéinase neutre activée par le calcium (CANP) pour la fabrication d'un contraceptif.

5. Utilisation selon n'importe laquelle des revendications 1 à 4, l'inhibiteur étant un inhibiteur endogène natif provenant d'une source biologique, sélectionnée parmi les hématies, le cerveau, le muscle cardiaque, le poumon, la rate, le foie, les muscles du squelette, les reins ou les testicules, et purifié, et comprenant des protéines tétramériques d'un PM d'environ 240 000, sur la base de leur élution à partir de Sephadex G-200, qui sont thermostables à un pH neutre ; détruites par digestion avec la trypsine, et dissociées en leurs sous-unités d'un PM d'environ 60 000 ou moins par 0,1 à 1 mM de $Ca^{2+}$, sur la base d'une électrophorèse sur gel SDS-polyacrylate, ou leurs sels d'addition pharmaceutiquement acceptables.

6. Utilisation selon la revendication 5, la source biologique de l'inhibiteur étant sélectionnée parmi les muscles squelettiques ou le foie du lapin.

7. Utilisation selon la revendication 6, l'inhibiteur provenant des muscles squelettiques du lapin.

8. Utilisation selon les revendications 1 à 4, les inhibiteurs des revendications 5 à 7 ou leurs sels d'addition pharmaceutiquement acceptables étant produits par synthèse.

9. Utilisation d'un inhibiteur spécifique selon n'importe laquelle des revendications 1 à 8 dans la préparation d'une préparation pharmaceutique qui est sous la forme d'une solution, d'une poudre, d'une injection, d'un comprimé, d'un gélule, d'une pastille, ou sous une forme à libération rapide ou prolongée.